(19) Europäisches Patentamt
European Patent Office
Office européen des brevets

(11) EP 3 936 113 A1

(12) EUROPEAN PATENT APPLICATION

(43) Date of publication:
**12.01.2022 Bulletin 2022/02**

(51) Int Cl.:
*A61K 9/00* (2006.01) *A61K 9/16* (2006.01)
*A61K 9/19* (2006.01) *A61K 9/50* (2006.01)

(21) Application number: **20305777.3**

(22) Date of filing: **07.07.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Occlugel**
**78350 Jouy en Josas (FR)**

(72) Inventors:
- **BEILVERT, Anne**
**91300 MASSY (FR)**
- **CORUS, Emeline**
**91640 JANVRY (FR)**
- **BEDOUET, Laurent**
**72000 LE MANS (FR)**
- **MOINE, Laurence**
**92210 SAINT CLOUD (FR)**

(74) Representative: **Regimbeau**
**20, rue de Chazelles**
**75847 Paris Cedex 17 (FR)**

# (54) HYDROPHILIC DEGRADABLE MICROSPHERE FOR DELIVERING TRAVOPROST

(57) The invention relates to a composition comprising an effective amount of a prostaglandin analogue, at least one hydrophilic degradable microsphere comprising a crosslinked matrix, and a pharmaceutically acceptable carrier for administration by injection, the crosslinked matrix being based on at least a) between 10 % and 90 % of hydrophilic monomer of general formula (I); b) between 0.1 and 30% mol of a cyclic monomer having an exo-methylene group of formula (II); and c) between 5 % and 90 % of one degradable block copolymer cross-linker, wherein the degradable block copolymer crosslinker is linear or star-shaped and presents ($CH_2$=($CR_{11}$))-groups at all its extremities. The invention also relates to such a composition for use for preventing and/or treating moderate to ocular hypertension or glaucoma.

EP 3 936 113 A1

## Description

## FIELD OF THE INVENTION

The present invention relates to hydrophilic degradable microsphere for delivering a prostaglandin analogue. In particular, the present invention relates to composition comprising an effective amount of prostaglandin analogues and hydrophilic degradable microspheres. The present invention also relates to said composition for use for preventing and/or treating ocular hypertension or glaucoma.

## TECHNICAL BACKGROUND

Glaucoma is a disease that damages the eye's optic nerve leading to progressive, irreversible vision loss. Glaucoma usually happens when fluid builds up in the front part of the eye. That extra fluid increases the pressure in the eye, inducing cell death of retinal ganglion cell neurons and their axons damaging the optic nerve over time. The damages created are irreversible, glaucoma is the second cause of blindness in the world. Actually around 60 million people are affected worldwide while 100 million are forecast in 2040 (Yadav 2019, Materials Science εt Engineering C : 103). The economic burden of glaucoma is important, with an overall cost to Medicare of $748 million in 2009 (Lambert 2015, Transl Vis Sci Technol. 4(1)). Annual eye care-related costs for glaucoma patients with no vision loss were $8157 (2007 US dollars); this increased to $14,237 for moderate to severe vision loss before reaching $18,670 for patients blinded by the disease.

The two main types of glaucoma are primary open-angle glaucoma and angle-closure glaucoma. Primary open-angle glaucoma is the most common form of the disease. The drainage angle formed by the cornea and iris remains open, but the trabecular meshwork is partially blocked. This leads to a gradual increase in pressure in the eye. This pressure damages the optic nerve and may lead to vision loss without signs or symptoms.

Angle-closure glaucoma, also called closed-angle glaucoma, occurs when the iris swells forward to narrow or block the drainage angle formed by the cornea and iris. As a result, fluid cannot circulate through the eye and pressure increases. Angle-closure glaucoma may occur suddenly (acute angle-closure glaucoma) or gradually (chronic angle-closure glaucoma).

If treated early, the progression of glaucoma can be slowed or stopped with medication, laser treatment, or surgery. The goal of these treatments is to reduce intraocular pressure, since it is impossible to repair the optic nerve. Eye drops are the treatment of choice for primary open-angle glaucoma. Treatments are therefore aimed to increase the elimination of aqueous humor, decrease its production or both. Reduction of aqueous humor production is the therapeutic goal achieved with carbonic anhydrase inhibitors (Brinzolamide, Dorzolamide) and β-blockers (Timolol, Levobunolol, Betaxolol, Carteolol). Other medications accelerate its elimination such as prostaglandin analogues (latanoprost, Bimatoprost travoprost).

However, problems with patient adherence to topical medications hinder glaucoma therapy with eyes drops. Regular application of the treatment is essential, as it is assumed that 1 mm Hg decrease in intraocular pressure can reduce the progression of glaucoma by 10%. Discontinuation of treatment after 6 months is reported in 50 % of patients (Nordstrom et al; 2005. Am J Ophthalmol. 140:598-606) while the persistence rate of patients drops to 22.5% after one year and 11.5 % three years after the first prescription (Quek et al; 2011. Arch Ophthalmol. 129:643-8). Further, a number of patients (13 %) fail to use eye drops correctly (Brown et al; 1984. Can J Ophthalmol. 19: 2-5). A poor adherence to treatment induces glaucoma progression in 50% of patients (Lambert 2015, Transl Vis Sci Technol. 4(1)).

Non-adherence to glaucoma medications via eye drops may be a major cause of treatment failure. Novel therapeutic solutions need to be developed to allow the daily application of anti-glaucomatous drugs. One of them is the subconjunctival injection of drug delivery systems (DDS) for sustained local delivery. One study in Singapore found that 74% of glaucoma patients were willing to accept an alternative form of glaucoma treatment through 3-monthly subconjunctival injections (Song et al; 2013. J Glaucoma. 22:190-4).

Subconjunctival injection in humans is a safe technique commonly used for various indications: injection of mitomycin C to decrease intraocular pressure (Gandolfi et al; 1995. Arch Ophthalmol. 113:582-5), corticosteroid injection for the treatment of anterior scleritis (Sen et al; 2005. Br J Ophthalmol. 89: 917-8) or macular oedema (Carbonnière et al; 2017. J Fr Ophtalmol. 40:177-86). The conjunctiva covers the anterior sclera, the bulbar conjunctiva, and lines the inner side of the eyelids, the palpebral conjunctiva. The conjunctiva is a thin, transparent membrane composed of an epithelium and stroma layers. The average total thickness of the human bulbar conjunctiva is around 240 μm, with an epithelium thickness of ≈ 50 μm and a stroma thickness of ≈190 μm (Zhang et al; 2011. Invest Ophthalmol Vis Sci. 52:7787-91). The subconjunctival injections occurred between the bulbar conjunctiva and the sclera. The injected volumes are usually between 0.1 and 0.5 mL (Subrizi et al; 2019, Drug Discovery Today, 24: 1446-57).

Different compositions were investigated for sustained drug delivery after single subconjunctival injection. Their delivery performances were evaluated *in vitro* and *in vivo* in rabbit or monkey. Several DDS were prepared with timolol

maleate (a beta-blocker). Timolol maleate was incorporated in microfilm implants (4 x 6 mm) with a thickness of 40 μm made of a copolymer of poly(lactide-co-caprolactone). Then, in monkeys after a limited conjunctival dissection, one timolol loaded microfilm was inserted before suture of the conjunctiva. A sustained intraocular pressure (IOP) reduction was observed for 5 months (Ng et al; 2015. Drug Deliv. and Transl. Res. 5:469-79). Encapsulation of timolol maleate was also done in PLGA degradable microspheres (mean diameter of 14 μm). The sustained delivery of timolol occurred for 3 months *in vitro,* and after subconjuntival injection in ocular normotensive rabbit, a sustained IOP-lowering effect was measured for 3 months (Lavik et al; 2016. J Ocul Pharmacol Ther. 32:642-49). Huang et al. (J Ocul Pharmacol Ther. 21:445-53; 2005) prepared timolol maleate discs composed of PLGA which reduce IOP only during one week after implantation onto the cul de sac of hypertensive rabbits. One other β-blocker, the brimonidine tartrate was incorporated in PLGA microspheres (average diameter of 7.4 μm). After a single subconjunctival injection in rabbit (150 μL of MS suspension), an ocular hypotensive effect was measured during one month (Fedorchak et al ; 2014. Exp Eye Res. 2014 Aug; 125:210-6).

Brinzolamide, a carbonic anhydrase inhibitor which decreases the secretion of aqueous humor, was encapsulated in nanoparticles of PLGA. Their subconjunctival injection in normotensive rabbits triggers a reduction for 10 days (Salama et al; 2017. AAPS PharmSciTech. 18 : 2517-28).

Injectable DDS for sustained delivery of prostaglandin analogues for subconjunctival injections were described. Giarmoukakis et al (Exp Eye Res. 112:29-36; 2013) reported preparation of PLA-PEG nanoparticles (80 nm size) containing latanoprost. In vitro, the release was achieved after one week, and in vivo after minimally invasive subconjunctival injection, a significant hypotensive effect for up to 8 days was obtained in normotensive rabbit. Latanoprost was also encapsulated in liposomes of 100 nm. In vitro, the drug release was sustained for 1 month, and after a single subconjunctival injection in non-human primate, a sustained IOP-lowering effect was observed for 4 months (Natarajan et al; 2014. ACS Nano. 8: 419-29).

Preclinical studies demonstrate that a single subconjunctival implantation of different DDS such degradable microfilms, microspheres, liposomes or nanoparticles are efficient to reduce the intraocular pressure for several weeks or months. This route of administration of hypotensive drug incorporated in DSS seems efficient. Some of the DDS described above are inappropriate for clinical use, such the solid implants of PLGA which require an incision of conjunctiva for their implantation. The others DDS (liposomes, nanospheres, microspheres) can be implanted min-invasively by injection. Most of them have a size lower than the diameter of the blood vessel present in the bulbar conjunctiva (between 16 and 22 μm) (Shu et al. 2019. Eye and Vision. 6:15). During subconjunctival injection a risk of accidental embolization of retinal and choroidal vessels cannot be excluded. Case of central retinal artery occlusion are described after peribulbar injections of steroids which contain crystals between 1 μm to 1000 μm (Li et al; 2018. Medicine 97:17; Benzon et al; 2007. Anesthesiology. 106: 331-8). The mechanism is based on inattentive intra-arterial injection of corticoids and due to the diffuse anastomoses of the facial arterial system, facial injection might cause retrograde embolization with glucocorticoid crystals of ophthalmic or central retinal arteries leading to vision loss. To avoid such accident, after subconjunctival injection, the size and the geometry of DDS containing the anti-glaucoma drugs appears to be key parameters. Ideally, their size should be larger than the diameter of the blood vessels in the conjunctival tissue, but they should be flexible enough to be injected through thin needles.

To locally treat glaucoma the inventors have noticed the interest represented by the local degradable delivery systems.

## SUMMARY OF THE INVENTION

The inventors surprisingly found strong interaction between prostaglandin analogues such as travoprost with hydrophilic degradable microspheres of size range 50-100 μm composed of a crosslinked hydrogel.

Prostaglandin analogues are a class of drugs that bind to a prostaglandin receptor. Prostaglandin analogues are used for the treatment of most forms of glaucoma. The compounds should be used whenever low target pressures are called for in both normal-tension glaucoma or primary open-angle glaucoma (POAG), as well as ocular hypertensive (OHT) patients where treatment seems mandatory. The most commonly known prostaglandin analogues are travoprost, latanoprost, bimatoprost and tafluprost.

Travoprost, a prostaglandin analogue, is used to treat open angle glaucoma when other agents are not sufficient. Travoprost is a synthetic analogue of prostaglandin F2α that works by increasing the outflow of aqueous fluid from the eyes. Travoprost concentration of eye drops solutions is 40 μg/mL, and according to a dosage of 1 drop (≈ 50 μL) in the conjunctival sac of the eye once a day, approximatively 2 μg of travoprost are applied to the cornea every day. A local DDS of travoprost must be able to release this amount every day for several months.

The present invention offers the possibility to tune the amount of loaded prostaglandin analogues such as travoprost (25-50-fold higher than commercial topical travoprost) and the flow rate of prostaglandin analogues such as travoprost release during the time. In addition, the relatively large diameter of the microsphere compared to the anterior art would minimize the retrograde occlusion danger of the retina and of the choroidal vessels during the subconjunctival

injection.

The inventors have discovered hydrophilic degradable microspheres that may be used as biocompatible drug carrier for peri-ocular drug delivery and that present affinity with the active ingredient prostaglandin analogues such as travoprost, latanoprost, bimatoprost and tafluprost, in particular travoprost.

The inventors have thus discovered a prostaglandin analogues delivery system that is free of organic solvent, that presents a tuneable degradation time from day to months, that is easy to load (in water, at room temperature), that allows a long-term drug release and that avoids intense inflammatory reaction.

In a first aspect, the invention relates to a composition comprising an effective amount of a prostaglandin analogue, at least one hydrophilic degradable microsphere comprising a crosslinked matrix, and a pharmaceutically acceptable carrier for administration by injection, the crosslinked matrix being based on at least:

a) between 10 % and 90 % of hydrophilic monomer of general formula (I):

$$(CH_2{=}CR_1)\text{-}CO\text{-}D \qquad (I)$$

wherein:

- D is O-Z or NH-Z, Z being $(C_1\text{-}C_6)$alkyl group, $-(CR_2R_3)_m\text{-}CH_3$, $-(CH_2\text{-}CH_2\text{-}O)_m\text{-}H$, $(CH_2\text{-}CH_2\text{-}O)_m\text{-}CH_3$, $-C(R_4OH)m$ or $-(CH_2)_m\text{-}NR_5R_6$ with m being an integer from 1 to 30;
- $R_1$, $R_2$, $R_3$, $R_4$, $R_5$ and $R_6$ are, independently of one another, hydrogen atom or a $(C_1\text{-}C_6)$alkyl group;

b) between 0.1 and 30% mol of a cyclic monomer having an exo-methylene group of formula (II):

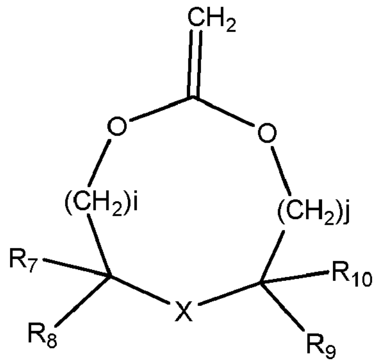
(II)

wherein:

- $R_7$, $R_8$, $R_9$ and $R_{10}$ are, independently of one another, hydrogen atom, a $(C_1\text{-}C_6)$alkyl group or an aryl group;
- i and j are independently of one another an integer chosen between 0 and 2; and
- X is a single bond or an oxygen atom;

and

c) between 5 % and 90 % of one degradable block copolymer cross-linker, wherein the degradable block copolymer crosslinker is linear or star-shaped and presents $(CH_2{=}(CR_{11}))$-groups at all its extremities, each $R_{11}$ being independently of one another hydrogen atom or a $(C_1\text{-}C_6)$alkyl group,

the percentages of the monomers a) to c) being expressed in moles relative to the total number of moles of the monomers.

In a second aspect, the invention relates to the composition of the invention, for use for preventing and/or treating ocular hypertension or glaucoma.

In a third aspect, the invention relates to the hydrophilic degradable microsphere of the invention for use for delivering an effective amount of a prostaglandin analogue, advantageously of travoprost, to a subject in need thereof.

In a fourth aspect, the invention relates to a pharmaceutical kit comprising:

i) at least one hydrophilic degradable microsphere of the invention in association with a pharmaceutically acceptable carrier for administration by injection;
ii) an effective amount of a prostaglandin analogue, advantageously of travoprost; and
iii) optionally an injection device,

the hydrophilic degradable microsphere and the travoprost being packed separately.

## DETAILED DESCRIPTION OF THE INVENTION

*Definition*

In the context of the invention, by the expression "matrix based on" is meant a matrix comprising the mixture and/or the product of the reaction between the starting components used for the heterogeneous medium polymerisation of this matrix, preferably only the product of the reaction between the different starting components used for this matrix, some of which may be intended to react or may react with each other or with their close chemical environment, at least in part, during the different phases of the process of manufacture of the matrix, in particular during a polymerisation stage. Thus, the starting components are the reagents intended to react together during the polymerization of the matrix. The starting components are therefore introduced into a reaction mixture optionally additionally comprising a solvent or a mixture of solvents and/or other additives such as at least one salt and/or at least one polymerization initiator and/or at least one stabilizer such as PVA.
In the context of the present invention, the reaction mixture comprises at least the monomers a), b), c) as defined in the claims and in this description, optionally a polymerization initiator such as, for example, t-butyl peroxide, benzoyl peroxide, azobiscyanovaleric acid (also called 4,4'-Azobis(4-cyanopentanoic acid)), AIBN (azobisisobutyronitrile), or 1,1'- Azobis(cyclohexane carbonitrile) or one or more photo-initiators such as 2-Hydroxy-4'-(2-hydroxyethoxy)-2-methylpropiophenone (106797-53-9); 2-Hydroxy-2-methylpropiophenone (Darocur® 1173, 7473-98-5); 2,2-Dimethoxy-2-phenylacetophenone (24650-42-8); 2,2-Dimethoxy-2-phenyl acetophenone (Irgacure®, 24650-42-8) or 2-Methyl-4'-(methylthio)-2-morpholinopropiophenone (Irgacure®, 71868-10-5), and at least one solvent, preferably a solvent mixture comprising an aqueous solvent and an organic solvent such as an apolar aprotic solvent, for example a water/toluene mixture.
Thus, according to the present invention, the matrix is at least based on the monomers a), b) and c) as defined in the claims and in this description, these compounds thus being starting components.

Thus, in the present description, expressions such as "the [starting component X] is added to the reaction mixture in an amount of between YY% and YYYY%" and "the cross-linked matrix is based on [starting component X] in an amount of between YY% and YYYY%" are interpreted in a similar manner. Similarly, expressions such as "the reaction mixture comprises at least [starting component X]" and "the cross-linked matrix is based on at least [starting component X]" are interpreted in a similar manner.

In the context of the invention, "organic phase" of the reaction mixture means the phase comprising the organic solvent and the compounds soluble in said organic solvent, in particular the monomers, and the polymerization initiator.

In the context of the invention, the terms "($C_X$-$C_Y$)alkyl group" mean a saturated monovalent hydrocarbon chain, linear or branched, containing X to Y carbon atoms, X and Y being integers between 1 and 36, preferably between 1 and 18, in particular between 1 and 6. Examples are methyl, ethyl, propyl, isopropyl, butyl, isobutyl, sec-butyl, tert-butyl, pentyl or hexyl groups.

In the context of the invention, the terms "aryl group" and "($C_X$-$C_Y$)aryl" mean an aromatic hydrocarbon group, preferably having X to Y carbon atoms, and comprising one or more adjacent rings, X and Y being integers between 5 and 36, preferably between 5 and 18, in particular between 5 and 10. Examples are phenyl or naphthyl groups.

In the context of the invention, the terms "partition coefficient P" mean the ratio of concentrations of a compound in a mixture of two immiscible solvents at equilibrium: water and 1-octanol. This ratio is therefore a comparison of the solubilities of the solute in these two liquids. Hence the octanol/water partition coefficient measures how hydrophilic (octanol/water ratio $< 1$) or hydrophobic (octanol/water $> 1$) a compound is. Partition coefficient P may be determined by measuring the solubilities of the compound in water and in 1-octanol and by calculating the ratio solubility in octanol / solubility in water. Partition P may also be determined *in silico.*

In the context of the invention, the hydrophobic/hydrophilic balance R of the degradable crosslinkers is quantified by the ratio of the number of hydrophobic units to the number of hydrophilic units according the following equation:

$$R = \frac{N_{hydrophobic\ units}}{N_{hydrophilic\ units}}$$

with N being an integer and representing the number of unit(s).

For example, for the crosslinkers that can be used in the present invention, R is:

$$R = \frac{N_{CHlactide} + N_{CH3lactide} + N_{CH2glycolide} + 5 \times N_{CH2caprolactone}}{N_{EO\ unit}}$$

with N being an integer and representing the number of unit(s).

In the context of the invention, the terms "degradable microsphere" mean that the microsphere is degraded or cleaved by hydrolysis in a mixture of degradation products composed of low-molecular-weight compounds and water-soluble polymer chains having molecular weights below the threshold for renal filtration of 50 kg $mol^{-1}$. Compared to non-degradable microspheres, no ester hydrolysable bonds is present within the crosslinker.

In the context of the invention, the expression "between X and Y" (wherein X and Y are numerical value) means a range of numerical values in which the limits X and Y are inclusive.

In the context of the invention, the expression "immediate release (IR)" of an active ingredient means the rapid release of the active ingredient from the formulation to the location of delivery as soon as the formulation is administered.

In the context of the invention, the expression "extended-release" of an active ingredient means either the "sustained-release (SR)" or the "controlled-release (CR)" of active ingredients from the formulation to the location of delivery at a predetermined rate for an extended period of time and maintaining a constant active ingredient level for this period of time with minimum side effects. The controlled-release (CR) differs from the sustained-release (SR) in that CR maintains drug release over a sustained period at a constant rate whereas SR maintains drug release over a sustained period but not at a constant rate.

In the context of the invention, the expression "sustained-release" of an active ingredients means an extended-release (as defined above) of an active ingredient from the formulation to the location of delivery in order to maintain for a certain predetermined time the drug in tissue of interest at therapeutic concentrations by means of an initial dose portion.

In the context of the invention, the expression "controlled-release (CR)" of an active ingredient means an extended-release (as defined above) of an active ingredient from the formulation to the location of delivery, that provides some control of temporal or spatial nature, or both.

For the purpose of the invention, the term "pharmaceutically acceptable" is intended to mean what is useful to the preparation of a pharmaceutical composition, and what is generally safe and non toxic, for a pharmaceutical use. The term « pharmaceutically acceptable salt » is intended to mean, in the framework of the present invention, a salt of a compound which is pharmaceutically acceptable, as defined above, and which possesses the pharmacological activity of the corresponding compound. Such salts comprise:

(1) hydrates and solvates,
(2) acid addition salts formed with inorganic acids such as hydrochloric, hydrobromic, sulfuric, nitric and phosphoric acid and the like; or formed with organic acids such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L-tartaric, tartaric, p-toluenesulfonic, trimethylacetic, and trifluoroacetic acid and the like, and
(3) salts formed when an acid proton present in the compound is either replaced by a metal ion, such as an alkali metal ion, an alkaline-earth metal ion, or an aluminium ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate and sodium hydroxide.

*Microsphere*

According to the present invention, the hydrophilic degradable microsphere comprises a crosslinked matrix that is based on at least:

a) between 10 % and 90% of hydrophilic monomer of general formula (I):

$$(CH_2{=}CR_1)\text{-}CO\text{-}D \qquad (I)$$

wherein:

- D is O-Z or NH-Z, Z being $(C_1\text{-}C_6)$alkyl group, $-(CR_2R_3)_m\text{-}CH_3$, $-(CH_2\text{-}CH_2\text{-}O)_m\text{-}H$, $(CH_2\text{-}CH_2\text{-}O)_m\text{-}CH_3$, $-C(R_4OH)_m$ or $-(CH_2)_m\text{-}NR_5R_6$ with m being an integer from 1 to 30;
- $R_1$, $R_2$, $R_3$, $R_4$, $R_5$ and $R_6$ are, independently of one another, hydrogen atom or a $(C_1\text{-}C_6)$alkyl group;

b) between 0.1 and 30% mol of a cyclic monomer having an exo-methylene group of formula (II):

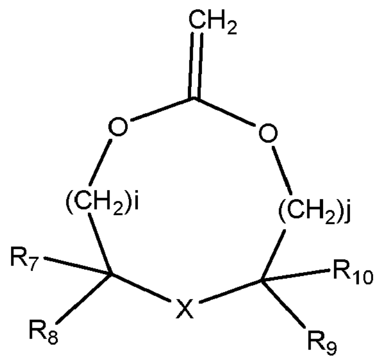

(II)

wherein:

- $R_7$, $R_8$, $R_9$ and $R_{10}$ are, independently of one another, hydrogen atom, a $(C_1-C_6)$alkyl group or an aryl group;
- i and j are independently of one another an integer chosen between 0 and 2; and
- X is a single bond or an oxygen atom;

and

c) between 5 % and 90 % of one degradable block copolymer cross-linker, wherein the degradable block copolymer crosslinker is linear or star-shaped and presents $(CH_2=(CR_{11}))$-groups at all its extremities, each $R_{11}$ being independently of one another hydrogen atom or a $(C_1-C_6)$alkyl group, and wherein the degradable block copolymer crosslinker has a partition coefficient P of between 0.50 and 11.20, or a hydrophobic/hydrophilic balance R between 1 and 20;

the percentages of the monomers a) to c) being expressed in moles relative to the total number of moles of the monomers.

The components a) the hydrophilic monomer of general formula (I), b) the cyclic monomer having an exo-methylene group of formula (II), and c) the degradable block copolymer cross-linker, are thus starting components of the crosslinked matrix.

In the context of the invention, the hydrophilic degradable microsphere is a swellable degradable (i.e. hydrolyzable) cross-linked polymer in the form of spherical particle having a diameter after swelling ranging between 20 μm and 1200 μm. In particular the polymer of the invention is constituted of at least one chain of polymerized monomers a), b) and c) as defined above.

In the context of the invention, a polymer is swellable if it has the capacity to absorb liquids, in particular water. The expression "size after swelling" means thus that the size of the microspheres is considered after the polymerization and sterilization steps that take place during their preparation.

Advantageously, the microsphere of the invention has a diameter after swelling of between 20 μm and 100 μm, 40 μm and 150 μm, 100 μm and 300 μm, 300 μm and 500 μm, 500 μm and 700 μm, 700 μm and 900 μm or 900 μm and 1200 μm, advantageously of between 20 μm and 100 μm, 40 μm and 150 μm, 100 μm and 300 μm, 300 μm and 500 μm, 500 μm and 700 μm as determined by optical microscopy. Microspheres are advantageously small enough in diameter to be injected through needles, catheters or microcatheters with internal diameters ranging from a few hundred micrometres to more than one millimetres.

In the context of the invention, the terms "hydrophilic monomer" mean a monomer having a high affinity for water, i.e. tending to dissolve in water, to mix with water, to be wetted by water, or capable of swelling in water after polymerization.

The hydrophilic monomer a) is of general formula (I):

$$(CH_2=CR_1)\text{-CO-D} \qquad (I)$$

wherein:

- D is O-Z or NH-Z, Z being $(C_1-C_6)$alkyl group, $-(CR_2R_3)_m-CH_3$, $-(CH_2-CH_2-O)_m-H$, $(CH_2-CH_2-O)_m-CH_3$, $-C(R_4OH)_m$ or $-(CH_2)_m-NR_5R_6$ with m being an integer from 1 to 30;
- $R_1$, $R_2$, $R_3$, $R_4$, $R_5$ and $R_6$ are, independently of one another, hydrogen atom or a $(C_1-C_6)$alkyl group.

Advantageously, the hydrophilic monomer a) is selected from the group consisting of sec-butyl acrylate, n-butyl

acrylate, t-butyl acrylate, t-butyl methacrylate, methylmethacrylate, N-dimethylaminoethyl(methyl)acrylate, N,N-dimethylaminopropyl-(meth)acrylate, t-butylaminoethyl (methyl)acrylate, N,N-diethylaminoacrylate, acrylate terminated poly(ethylene oxide), methacrylate terminated poly(ethylene oxide), methoxy poly(ethylene oxide) methacrylate, butoxy poly(ethylene oxide) methacrylate, acrylate terminated poly(ethylene glycol), methacrylate terminated poly(ethylene glycol), methoxy poly(ethylene glycol) methacrylate, butoxy poly(ethylene glycol) methacrylate; advantageously acrylate terminated poly(ethylene glycol), methacrylate terminated poly(ethylene glycol), methoxy poly(ethylene glycol) methacrylate, butoxy poly(ethylene glycol) methacrylate..

More advantageously, the hydrophilic monomer a) is poly(ethylene glycol) methyl ether methacrylate.

In the context of the invention, the hydrophilic monomer a) is advantageously present in the reaction mixture in an amount of between 10% and 90%, preferably from between 30% and 80 % by mole, relative to the total number of moles of the monomers.

In the context of the invention, component b) is a cyclic monomer having an exo-methylene group of formula (II) as defined above, wherein:

- $R_7$, $R_8$, $R_9$ and $R_{10}$ are, independently of one another, hydrogen atom, a $(C_1-C_6)$alkyl group or an aryl group;
- i and j are independently of one another an integer chosen between 0 and 2;
- X is a single bond or an oxygen atom.

Advantageously, component b) is a cyclic monomer having an exo-methylene group of formula (II) as defined above, wherein:

- $R_7$, $R_8$, $R_9$ and $R_{10}$ are, independently of one another, hydrogen atom or a $(C_5-C_7)$aryl group;
- i and j are independently of one another an integer chosen between 0 and 2;
- X is a single bond or an oxygen atom.

Advantageously, component b) is a cyclic monomer having an exo-methylene group of formula (II) as defined above, wherein:

- $R_7$, $R_8$, $R_9$ and $R_{10}$ are, independently of one another, hydrogen atom or a $(C_5-C_7)$aryl group;
- i and j are independently of one another an integer chosen between 0 and 1X is a single bond or an oxygen atom.

Advantageously, the component b) is selected from the group consisting of 2-methylene-1,3-dioxolane, 2-methylene-1,3-dioxane, 2-methylene-1,3-dioxepane, 2-Methylene-1,3,6-Trioxocane and derivatives thereof, in particular benzo derivatives and phenyl substituted derivatives, advantageously from the group consisting of 2-methylene-1,3-dioxolane, 2-methylene-1,3-dioxane, 2-methylene-1,3-dioxepane, 2-methylene-4-phenyl-1,3-dioxolane, 2-Methylene-1,3,6-Trioxocane and 5,6-benzo-2-methylene-1,3dioxepane, more advantageously from the group consisting of 2-methylene-1,3-dioxepane, 5,6-benzo-2-methylene-1,3dioxepane and 2-Methylene-1,3,6-Trioxocane. More advantageously, the component b) is 2-methylene-1,3-dioxepane or 2-Methylene-1,3,6-Trioxocane.

In the context of the invention, the cyclic monomer b) having an exo-methylene group of general formula (II) is advantageously present in the reaction mixture in an amount of between 0.1 % and 30 %mol, preferably from between 1% and 20 %, and in particular between 1% and 10% by mole, relative to the total number of moles of the monomers.

In the context of the invention, component c) is a degradable block copolymer crosslinker, wherein the degradable block copolymer crosslinker is linear or star-shaped and presents $(CH_2=(CR_{11}))$-groups at all its extremities, each $R_{11}$ being independently of one another hydrogen atom or a $(C_1-C_6)$alkyl group.

In the context of the invention, the degradable block copolymer crosslinker has a partition coefficient P of between 0.50 and 11.20, advantageously between 3.00 and 9.00. Or the degradable block copolymer crosslinker has a hydrophobic/hydrophilic balance R between 1 and 20, advantageously between 3 and 15.

As intended therein, the expression "copolymer cross-linker" is intended to mean that the copolymer contains a functional group containing a double bond at least two of its extremities in order to link together several polymer chains.

The cross-linker c) as defined above is linear or star-shaped (advantageously from 3 to 8 arms) and it presents $(CH_2=(CR_{11}))$-groups at all its extremities, each $R_{11}$ being independently of one another hydrogen atom or a $(C_1-C_6)$alkyl group. Advantageously, the crosslinker c) presents $(CH_2=(CR_{11}))$-CO- or $(CH_2=(CR_{11}))$-CO-O groups at all its extremities, each $R_{11}$ being independently of one another hydrogen atom or a $(C_1-C_6)$alkyl group. Advantageously, the $R_{11}$ are identical and are H or a $(C_1-C_6)$alkyl group.

Advantageously, the cross-linker c) as defined above is linear and it presents $(CH_2=(CR_{11}))$-groups at both its extremities, each $R_{11}$ being independently of one another hydrogen atom or a $(C_1-C_6)$alkyl group. Advantageously, the crosslinker c) presents $(CH_2=(CR_{11}))$-CO- or $(CH_2=(CR_{11}))$-CO-O groups at both its extremities, each $R_{11}$ being independently of one another hydrogen atom or a $(C_1-C_6)$alkyl group. Advantageously, the $R_{11}$ are identical and are H or a

$(C_1-C_6)$alkyl group.

Advantageously, the crosslinker c) is of general formula (IIIa), (IIIb) or (IIIc) as follows:

$$(CH_2=CR_{11})\text{-}CO\text{-}X_n\text{-}PEG_p\text{-}X_k\text{-}CO\text{-}(CR_{11}=CH_2) \quad \text{(IIIa);}$$

$$(CH_2=CR_{11})\text{-}CO\text{-}O\text{-}X_n\text{-}PEG_p\text{-}X_k\text{-}O\text{-}CO\text{-}(CR_{11}=CH_2) \quad \text{(IIIb)}$$

$$PEG_p\text{-}(X_n\text{-}O\text{-}CO\text{-}(CR_{11}=CH_2))_z \quad \text{(IIIc);}$$

wherein:

- each $R_{11}$ is independently of one another hydrogen atom or a $(C_1-C_6)$alkyl group;
- X independently represents PLA, PGA, PLGA, PCL or PLAPCL;
- n, k and p respectively represent the degree of polymerization of X, and PEG, n and k independently being integers from 1 to 150, and p being an integer from 1 to 100;
- z represent the number of arms of the PEG molecule being integers from 3 to 8.

Advantageously, when the crosslinker c) is of general formula (IIIc), n may be identical or different in each arm of the PEG.

In the context of the invention:

- PEG means polyethylene glycol of formula (IVa):

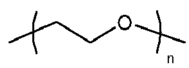
(IVa);

- PLA means poly-lactic acid (also named poly-lactide) of formula (IVb):

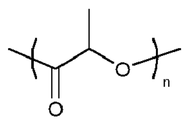
(IVb);

- PGA means poly-glycolic acid (also named poly-glycolide) of formula (IVc):

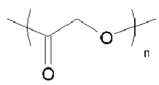
(IVc);

- PLGA means poly-lactic-glycolic acid and comprises both lactide and glycolide units, the degree of polymerization is the sum of the number of lactide and glycolide units; and
- PCL means poly(caprolactone) of formula (IVd):

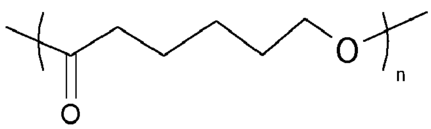
(IVd);

and

- PLAPCL means poly-lactic acid poly-caprolactone and comprises both lactide and caprolactone units, the degree of polymerization is the sum of the number of lactide and caprolactone units.

Advantageously, the crosslinker c) is of general formula (IIIa), (IIIb) or (IIIc), in particular (IIIa) or (IIIb), as defined above, wherein X represents PLAPCL or PCL. More advantageously, the crosslinker c) is of general formula (IIIa), (IIIb)

or (IIIc), in particular (IIIa) or (IIIb), wherein X represents PCL.

Advantageously, the crosslinker c) is of general formula (IIIa), (IIIb) or (IIIc), in particular (IIIa) or (IIIb), as defined above, wherein n and k independently being integers from 1 to 150, and p being an integer from 1 to 100.

Advantageously, the crosslinker c) is of general formula (IIIa), (IIIb) or (IIIc), in particular (IIIa) or (IIIb), as defined above, wherein the $R_{11}$ are identical and are H or a $(C_1-C_6)$alkyl group.

Advantageously, the crosslinker c) is selected from the group consisting of compounds of general formula (IIIa), (IIIb) or (IIIc), in particular (IIIa) or (IIIb), as defined above, wherein:

- X= PLA, n + k=12 and p=13 (such as PEG13-PLA12);
- X= PLAPCL, n + k=10 and p=13 (such as PEG13-LA8-Cl2 or PEG13-LA7-Cl3);
- X = PLAPCL, n + k= 9 and p=13 (such as PEG13-LA4-Cl5);
- X = PLAPCL, n + k= 8 and p=13 (such as PEG13-LA2-Cl6);
- X = PCL; n + k = 8 and p=13 (such as PEG13-PCl8);
- X =PCL, n + k = 10 and p=4 (such as PEG4-PCL10); or
- X =PCL, n + k = 12 and p=2 (such as PEG2-PCL12).

Within the definitions of the crosslinker c) above, the polyethylene glycol (PEG) has a length of 100 to 10 000 g/mol, preferably 100 to 2 000 g/mol, more preferably 100 to 1 000 g/mol.

In the context of the invention, the crosslinker c) is advantageously present in the reaction mixture in an amount of between 5 % and 90 %mol, preferably from between 5% and 60 % by mole, more preferably between 15 % and 60 % by mole, relative to the total number of moles of the monomers.

Increasing the amount of crosslinker, and thus decreasing the mesh size of the resulting microsphere, influences the loading of the microsphere in prostaglandin analogues, such as travoprost, and then the release of the prostaglandin analogues, such as travoprost. For amount greater than 15%, an optimal release of the travoprost is achieved, in particular because it prevents the immediate release of a large part of the travoprost.

According to the invention, the crosslinked matrix of the hydrophilic degradable microsphere is advantageously further based on a chain transfer agent d).

For the purposes of this invention, "transfer agent" means a chemical compound having at least one weak chemical bond. This agent reacts with the radical site of a growing polymer chain and interrupts the growth of the chain. In the chain transfer process, the radical is temporarily transferred to the transfer agent which restarts growth by transferring the radical to another polymer or monomer.

Advantageously, the chain transfer agent d) is selected from the group consisting of monofunctional or polyfunctional thiols, alkyl halides, transition metal salts or complexes and other compounds known to be active in free radical chain transfer processes such as 2,4-diphenyl-4-methyl-1-pentene. More advantageously, the chain transfer agent is a cycloaliphatic or aliphatic, thiol preferably having from 2 to 24 carbon atoms, more preferably between 2 and 12 carbon atoms, and having or not a further functional group selected from the groups amino, hydroxy and carboxy. Advantageously, the chain transfer agent d) is selected from the group consisting of thioglycolic acid, 2-mercaptoethanol, dodecane thiol and hexane thiol.

According to the invention, the chain transfer agent is advantageously present in the reaction mixture in an amount of, for example, from 0.1 to 10%, preferably from 2 to 5 % by mole, relative to the number of moles of monomer a).

In a particular aspect of the invention, the crosslinked matrix is only based on starting components a), b), c) and optionally d), as defined above and in the contents abovementioned, no other starting component are thus added to the reaction medium. It is thus clear that the sum of the above-mentioned contents of monomers (a), (b) and (c) must be equal to 100 %.

According to another aspect of the invention, the crosslinked matrix is advantageously further based on at least one ionised or ionisable monomer e) of general formula (V):

$$(CH_2{=}CR_{12})\text{-M-E} \quad (V),$$

wherein:

- $R_{12}$ is hydrogen atom or a $(C_1-C_6)$alkyl group;
- M is a single bond or a divalent radical having 1 to 20 carbon atoms, advantageously a single bond;
- E is a ionised or ionisable group being advantageously selected from the group consisting of -COOH, -COO-, $-SO_3H$, $-SO_3^-$, $-PO_4H_2$, $-PO_4H^-$, $-PO_4^{2-}$, $-NR_{13}R_{14}$, and $-NR_{15}R_{16}R_{17}^+$; $R_{13}$, $R_{14}$, $R_{15}$, $R_{16}$ et $R_{17}$ being independently of one another hydrogen atom or a $(C_1-C_6)$alkyl group.

In the context of the invention, an ionised or ionisable group is understood to be a group which is charged or

which may be in charged form (in the form of an ion), i.e. which carries at least one positive or negative charge, depending on the pH of the medium. For example, the COOH group may be ionised in the $COO^-$ form, and the $NH_2$ group may be ionised in the form of $NH_3^+$.

The introduction of an ionised or ionisable monomer into the reaction media makes it possible to increase the hydrophilicity of the resulting microspheres, thereby increasing the swelling rate of said microspheres, further facilitating their injection via catheters and microcatheters. In addition, the presence of an ionised or ionisable monomer improves the loading of active substances into the microsphere.

In an advantageous embodiment, the ionised or ionisable monomer e) is a cationic monomer, advantageously selected from the group consisting of -(methacryloyloxy)ethyl phosphorylcholine, 2-(dimethylamino)ethyl (meth)acrylate, 2-(diethylamino)ethyl (meth)acrylate and 2-((meth)acryloyloxy)ethyl] trimethylammonium chloride, more advantageously the cationic monomer is diethylamino)ethyl (meth)acrylate. Advantageously, the ionised or ionisable monomer e) is present in the reaction mixture in an amount of between 0 % and 30 % by mole, advantageously between 1 % and 30 % by mole, preferably from between 10% and 15 % by mole, relative to the total number of moles of the monomers.

In another advantageous embodiment, the ionised or ionisable monomer e) is an anionic monomer advantageously selected from the group consisting of acrylic acid, methacrylic acid, 2-carboxyethyl acrylate, 2-carboxyethyl acrylate oligomers, 3-sulfopropyl (meth)acrylate potassium salt and 2-(methacryloyloxy)ethyl]dimethyl-(3-sulfopropyl)ammonium hydroxide, more advantageously, the anionic monomer is acrylic acid. Advantageously, the ionised or ionisable monomer e) is present in the reaction mixture in an amount of between 0 % and 30 % by mole, advantageously between 1 % and 30 % by mole, preferably from between 10% and 15 % by mole, relative to the total number of moles of the monomers.

Advantageously, the ionised or ionisable monomer e) is acrylic acid and is advantageously present in the reaction mixture in an amount of between 0 % and 30 % by mole, advantageously between 1 % and 30 % by mole, preferably from between 10% and 15 % by mole, relative to the total number of moles of the monomers.

The microsphere of the invention can be readily synthesized by numerous methods well-known to the one skilled in the art. By way of example, the microsphere of the invention can be obtained by direct or inverse suspension polymerization as described below and in the Examples or by microfluidic.

A direct suspension may proceed as follows:

(1) stirring or agitating a mixture comprising

(i) at least the starting components a), b) and c) as defined above;
(ii) a polymerization initiator present in amounts ranging from 0.1 to approximately 2 parts per weight per 100 parts by weight of the monomers;
(iii) a surfactant in an amount no greater than about 5 parts by weight per 100 parts by weight of the monomers, preferably no greater than about 3 parts by weight and most preferably in the range of 0.5 to 1.5 parts by weight; and
(iv) water to form an oil in water suspension;

and

(2) polymerizing the starting components.

In such a direct suspension polymerization, the surfactant may be selected from the group consisting of hydroxyethylcellulose, polyvinyl alcohol (PVA), polyvinylpyrrolidone, polyethylene oxide, polyethylene glycol and Polysorbate 20 (Tween® 20).

An inverse suspension may proceed as follows:

(1) stirring or agitating a mixture comprising:

(i) at least the starting components a), b) and c) as defined above;
(ii) a polymerization initiator present in amounts ranging from 0.1 to approximately 2 parts per weight per 100 parts by weight of the monomers;
(iii) a surfactant in an amount no greater than about 5 parts by weight per 100 parts by weight of the monomers, preferably no greater than about 3 parts by weight and most preferably in the range of 0.5 to 1.5 parts by weight; and
(iv) oil to form a water in oil suspension;

and

(2) polymerizing the starting components.

In such a reverse suspension process, the surfactant may be selected from the group consisting of sorbitan esters such as sorbitan monolaurate (Span® 20), sorbitan monopalmitate (Span® 40), sorbitan monooleate (Span® 80), and sorbitan trioleate (Span® 85), hydroxyethyl cellulose, mixture of glyceryl stearate and PEG stearate (Arlacel®) and cellulose acetate.

In the above processes, the polymerization initiator may include t-butyl peroxide, benzoyl peroxide, azobiscyanovaleric acid (also known as 4,4'-Azobis(4-cyanopentanoic acid)), AIBN (azobisisobutyronitrile), or 1,1' Azobis (cyclohexane carbonitrile) or one or more photo-initiators such as 2-Hydroxy-4'-(2-hydroxyethoxy)-2-methylpropiophenone (106797-53-9); 2-Hydroxy-2-methylpropiophenone (Darocure 1173, 7473-98-5); 2,2-Dimethoxy-2-phenylacetophenone (24650-42-8); 2,2-dimethoxy-2-phenyl acetophenone (Irgacure®, 24650-42-8) or 2-Methyl-4'-(methylthio)-2-morpholinopropiophenone (Irgacure®, 71868-10-5).

Further, the oil may be selected from paraffin oil, silicone oil and organic solvents such as hexane, cyclohexane, ethyl acetate or butyl acetate.

Travoprost loading may proceed by numerous methods well-known to one of skill in the art such as passive adsorption (swelling of the polymer into a drug solution).

In order to increase the drug loading and control the rate of drug release, a concept consists to introduce certain chemical moieties into the polymer backbone that are capable of interacting with the drug via non covalent interactions. Examples of such interactions include electrostatic interactions (described above), hydrophobic interactions, $\pi$-$\pi$ stacking, and hydrogen bonding, among others.

*Drug*

In the context of the invention, the composition comprises an effective amount of a prostaglandin analogue such as travoprost, latanoprost, bimatoprost and tafluprost, in particular travoprost.

Advantageously, the prostaglandin analogue is selected form travoprost, latanoprost, bimatoprost and tafluprost. Advantageously, the prostaglandin analogue is travoprost.

Advantageously, in the composition of the invention, the prostaglandin analogues, in particular travoprost, is loaded/absorbed onto the microsphere as defined above by non-covalent interactions. This particular way of entrapping drugs or prodrugs is called physical entrapment.

Loading of a prostaglandin analogue, in particular travoprost, onto the microsphere of the invention may be proceeded by numerous methods well-known to the one skilled in the art such as preloading a prostaglandin analogue, in particular travoprost, after the microsphere synthesis.

Advantageously, the composition of the invention comprises between 1 and 6 mg/ml of a prostaglandin analogue, in particular travoprost, more advantageously between 2 and 4 mg/ml.

Advantageously, the composition of the invention releases the prostaglandin analogue, in particular the travoprost, without a burst, less 10 % during the first day, followed by a constant delivery rate between 1 % and 5 % of initial loading every day.

Advantageously, after implantation in living organisms, the composition of the invention releases the prostaglandin analogue, in particular the travoprost, in lachrymal fluid without a burst during the first hour following subconjunctival implantation. Concentration of the prostaglandin analogue, in particular travoprost, could remain in the therapeutic range in aqueous humor for 1 to 7 days, advantageously for 1 to 30 days, preferably for 1 to 90 days, the therapeutic range being between 2 ng/ml to 3 ng/ml (Martinez-de-la-Casa et al; 2012. Eye. 26: 972-75) or preferably with low plasma concentration, in the same range as observed after topical treatment (< 25 pg/mL) with eye-drops.

*Composition*

In the context of the invention, the composition comprises an effective amount of a prostaglandin analogue, such as travoprost, latanoprost, bimatoprost and tafluprost, in particular travoprost (0.1-0.6 % in mass relative to the microsphere), at least one hydrophilic degradable microsphere as defined above, and a pharmaceutically acceptable carrier for administration by injection.

The prostaglandin analogues, in particular the travoprost, and the hydrophilic degradable microsphere are as defined above.

According to the invention, the pharmaceutically acceptable carrier is intended for administration of a the prostaglandin analogue, in particular the travoprost, by injection and is advantageously selected in the group consisting in water for injection, saline, glucose, starch, hydrogel, polyvinylpyrrolidone, polysaccharide, hyaluronic acid ester, contrast agent and plasma.

The formulations may be administered by subconjunctival injection. The formulations of hydrophilic degradable

microspheres are syringable, the microsphere size and distribution are shown in Figure 5 for example. This enables the administration in a needle that is from between 21 and 34 gauge.

The composition of the invention can also contain a buffering agent, a preservative, a gelling agent, a surfactant, or mixtures thereof. Advantageously, the pharmaceutically acceptable carrier is saline or water for injection.

The composition of the invention allows the sustained release of the prostaglandin analogue, in particular travoprost, over a period ranging from a few hours to a few months. Advantageously, the composition of the invention allows the sustained-release of the prostaglandin analogue, in particular travoprost, for at least 4 weeks without burst, in particular between 4 weeks and 6 months, more particularly between 4 weeks and 3 months.

The composition of the invention allows the control of the sustained-release as defined above, for example by modulating the nature and the contents of monomers a), b) and/or c) and the amount of loaded the prostaglandin analogue, in particular travoprost.

The invention also relates to the composition as defined above, for use for preventing and/or treating ocular hypertension or glaucoma.

The invention also relates to a method for preventing and/or treating ocular hypertension or glaucoma, comprising administering to a subject in need thereof an effective amount of the composition as defined above.

The invention also relates to the use of the composition as defined above for the manufacturing of a drug for preventing and/or treating ocular hypertension or glaucoma.

*Extemporaneous loading*

In a particular embodiment of the invention, the travoprost may be loaded extemporaneously on dry and sterile microsphere.

The invention thus also relates to a pharmaceutical kit comprising:

i) at least one hydrophilic degradable microsphere as defined above in association with a pharmaceutically acceptable carrier for administration by injection;
ii) an effective amount of travoprost; and
iii) optionally an injection device,

the hydrophilic degradable microsphere and the travoprost being packed separately.
in such an embodiment, the travoprost is advantageously intended to be loaded on the hydrophilic degradable microsphere just before the injection.

According to the present invention, "injection device" means any device for parenteral administration. Advantageously, the injection device is one or more syringes, which may be pre-filled, and/or one or more catheters or microcatheters.

*Use of the microsphere*

The invention also relates to the hydrophilic degradable microsphere as defined above for use for the delivery, advantageously the sustained-delivery, of an effective amount of travoprost to a subject in need thereof.

Advantageously, the sustained delivery of travoprost is over a period ranging from a few weeks to a few months without burst, advantageously for at least 4 weeks, in particular between 4 weeks and 6 months, more particularly between 4 weeks and 3 months.

The composition of the invention allows the sustained release of travoprost over a period ranging from a few hours to a few months. Advantageously, the composition of the invention allows the sustained-release of travoprost for at least 4 weeks without burst, in particular between 4 weeks and 6 months, more particularly between 4 weeks and 3 months.

The examples which follow illustrate the invention without limiting its scope in any way.

**DESCRIPTION OF FIGURES**

Figure 1: In vitro release during 32 days of travoprost from MS5 after extemporaneous loading (5 min at room temperature) (1.98 mg/mL).

Figure 2 represents the effect of MS composition on travoprost release during the swelling of dry microspheres in saline during 10 minutes at room temperature. Effect of crosslinker composition and concentration on the travoprost burst during swelling of dry loaded microspheres. Comparisons between two groups of microspheres were done

using Mann-Whitney (MW) test. Significance was set at $p < 0.05$.

Figure 3: In vitro elution of travoprost during one month after loading at 1 mg/mL. The concentrations of crosslinker were at 5 % (MS3), 30 % (MS5) or 50 % (MS6)

Figure 4: In vitro elution of travoprost during one month after loading at 2 mg/mL. The concentrations of crosslinker were at 30 % (MS5) or 50 % (MS6)

Figure 5 : Size distribution of degradable microspheres MS1 and MS3

## EXAMPLES

### Example 1: Preparation of unloaded microsphere according to the invention

The starting components and their contents are summarized in Table 1. Table 1 also summarizes the main parameters.

| | | Microsphere of 50-100 µm diameters | | | | | | |
|---|---|---|---|---|---|---|---|---|
| | | **Test number** | **MS1** | **MS2** | **MS3** | **MS4** | **MS5** | **MS6** |
| Process parameters | | Oil/Water ratio (V/V) | 1/11 | 1/11 | 1/11 | 1/11 | 1/11 | 1/11 |
| | | Stirring speed | 240 RPM | 240 RPM | 240 RPM | 240 RPM | 240 RPM | 240 RPM |
| | | PVA | 1 % | 1 % | 1 % | 1 % | 1 % | 1 % |
| | | NaCl | 3 % | 3 % | 3 % | 3 % | 3 % | 3 % |
| Organic phase | | Monomers mass / organic phase mass (%) | 56 % | 56 % | 56 % | 56 % | 56 % | 56 % |
| | | Toluene | 44 % | 44 % | 44 % | 44 % | 44 % | 44 % |
| | | Hexanethiol (% mole / PEGma mole) | 3 % | 3 % | 3 % | 3 % | 3 % | 3 % |
| | | AIBN (% weight /weight organic phase | 0.28% | 0.28% | 0.28% | 0.28% | 0.28% | 0.28% |
| | Phase monomer | m-PEGMA (% mole / total mole monomer) | 85 % | 60 % | 85 % | 75 % | 60 % | 40 % |
| | | Crosslinker (% mole / total mole monomer) | 5 % of $PEG_{13}$-$LA_7$-$Cl_3$ | 30 % of $PEG_{13}$-$LA_7$-$Cl_3$ | 5 % of $PEG_{13}$-$PCl_8$ | 15 % of $PEG_{13}$-$PCl_8$ | 30 % of $PEG_{13}$-$PCl_8$ | 50 % of $PEG_{13}$-$PCl_8$ |
| | | 2-methylene-1,3-dioxepane (MDO) (% mole / total mole monomer) | 10 % | 10 % | 10 % | 10 % | 10 % | 10 % |

| | | | | | | | | |
|---|---|---|---|---|---|---|---|---|
| | | Methacrylic acid (% mole / total mole monomer) | 0 % | 0 % | 0 % | 0 % | 0 % | 0 % |

**Table 1. Formulations of microspheres according to the invention**

The aqueous phase solution (917 mL) containing 1 % polyvinyl alcohol (Mw= 13000-23000 g/mol), 3 % NaCl in deionized water is placed in a 1 $dm^3$ reactor and heated up to 50°C.

The organic phase is prepared in an Erlenmeyer. Briefly, toluene (36.9 g) and 2,2'-azobis(2-methylpropionitrile) (AIBN) (0.28% weight/ organic phase weight) are weighted. AIBN is introduced in another vial and solubilized in a volume fraction (≈ 30%) of the weighted toluene.

Then, degradable crosslinker is weighted in an Erlenmeyer. Polyethylene glycol methyl ether methacrylate (Mn= 300 g/mol) and 2-Methylene-1,3-dioxepane (MDO) are weighted and introduced into the erlenmeyer. Then the remaining volume of toluene is added to solubilize the monomers. Hexanethiol (3 % mol /mol of PEGma) is added to the Erlenmeyer. The AIBN solution in toluene is added to the erlenmeyer containing monomers. Finally, the organic phase has to be clear (monomer and initiator should be totally solubilized) without any aggregates before introduction into the aqueous phase.

The organic phase is poured into the aqueous phase at 50°C. Thereupon, stirring (240 rpm) is applied by using an impeller. After 4 minutes, the temperature is raised up to 80°C. After 8 hours, the stirring is stopped and microspheres were collected by filtration on a 40 μm sieve and washed extensively with acetone and water. Microspheres were then sieved with decreasing sizes of sieves (125, 100, 50 μm).

MS in the size range 50-100 μm were collected for drug loading trials.

**Example 2: Loading of microspheres according to example 1 with travoprost (preloading after MS synthesis)**

After the sieving step, 250 μL of microspheres obtained in example 1 (size range 50-100 μm) were placed in 15 mL polypropylene vials. Then, water (500 μL or 1 mL) was added, before the addition of 500 μL or 1 mL, respectively, of travoprost solution (Sigma, PHR 1622-3mL lot LRAA5292 (0.499 μg/mL in water/acetonitrile (70/30%)).

The loading step was done at room temperature for 1 h under stirring on a tube rotator (≈ 30 rpm). Then, the supernatants were removed for the measurement of unbound travoprost by fluorimetry ($\lambda_{ex}$ 220 nm, $\lambda_{Em}$ 310 nm). The amount of travoprost in supernatant was obtained by extrapolation from a standard curve (0.6 to 20 μg/mL), and the loaded dose was calculated by subtraction.

The pellets were washed with 2 mL of glucose (2.5 % in water). Then, the microsphere pellets were frozen-dried before e-beam sterilization (15 kilograys).

Table 2 summarizes travoprost loading for each MS formulation tested.

Table 2. Travoprost loading in the microspheres according to example 2

| Test number | Travoprost loading (mg/ml) for 0.5 mL of travoprost solution (% of loading efficacy) | Travoprost loading (mg/ml) for 1 mL of travoprost solution (% of loading efficacy) |
|---|---|---|
| MS1 | 0.93 ± 0.01 (93 %) | 1.79 ± 0.004 (90%) |
| MS2 | 0.98 ± 0.001 (99 %) | 1.95 ± 0.001 (97 %) |
| MS3 | 0.94 ± 0.004 (95 %) | 1.86 ± 0.0028 (93 %) |
| MS4 | 0.98 ± 0.0003 (99 %) | 1.95 ± 0.009 (98 %) |
| MS5 | 0.98 ± 0.003 (99 %) | 1.96 ± 0.001 (98 %) |
| MS6 | 0.99 ± 0.001 (99 %) | 1.97 ± 0.003 (98 %) |

**Example 3. Extemporaneous loading of travoprost on sterile microspheres according to example 1**

After the sieving step, a suspension of 250 μL of microspheres in 15 mL of a solution containing 2.5 % (w/v) of mannitol was prepared. After homogenization, the pellet of microspheres was recovered, frozen-dried and sterilized

by e-beam radiation (15-25 kilograys).

Then, 500 μL or 1 mL of water were added, before the addition of 500 μL or 1 mL, respectively, of travoprost solution (Sigma, PHR 1622-3mL lot LRAA5292 (0.499 μg/mL in water/acetonitrile (70/30%)).

The loading step was done at room temperature for 5 min under stirring on a tube rotator (≈ 30 rpm). Then, the supernatants were removed for the measurement of unbound travoprost by fluorimetry ($\lambda_{ex}$ 220 nm, $\lambda_{Em}$ 310 nm). The amount of travoprost was obtained by extrapolation from a standard curve (0.6 to 20 μg/mL).

Table 3 summarizes extemporaneous travoprost loading on dry and sterile MS of different formulations tested.

Table 3. Travoprost loading in the microspheres according to example 3

| Test number | Travoprost loading (mg/ml) for 0.5 mL of travoprost solution (% of loading efficacy) |
|---|---|
| MS1 | 0.9 ± 0.011 (90%) |
| MS5 | 1.98 ± 0.14 (99 %) |

Then, PBS (50 mL) was added to the microspheres loaded with travoprost for the *in vitro* drug release experiment (37°C, 150 rpm). The medium is completely renewed after each sample collection. The amounts of travoprost in PBS supernatants were determined by RP-HPLC at 222 nm on a $C_{18}$ column (46 x 150 mm) using a mobile phase made of acetonitrile/water containing 0.1 % TFA (60:40, v/v) at a flow rate of 1 mL/min in the isocratic mode at 25°C. The in vitro release from MS5 is shown in figure 1.

After extemporaneous loading on dry and sterile MS, the initial burst release of travoprost was slow (3.5 %) and sustained for at least one month.

**Example 4. Study of the *in vitro* release of travoprost from loading microsphere according to example 2**

For sterile and dry microspheres loaded with travoprost, the swelling step of microspheres was performed for 10 min in 10 mL of 0.9 % NaCl saline solution. After the removal of saline, 50 mL of PBS (Sigma P-5368; 10 mM phosphate buffered saline; NaCl 0,136 M; KCl 0,0027 M; pH 7.4 at 25°C) were added.
Drug elution occurred at 37°C under shaking (150 rpm), the tubes were placed horizontally in the oven. Samples (1 mL) were withdrawn after 2h, 24 h and every 3 or 4 days for one month. At each sampling time, the medium was completely renewed with fresh PBS.
The amounts of free travoprost in saline and PBS supernatants were determined by RP-HPLC at 222 nm on a $C_{18}$ column (46 x 150 mm) using a mobile phase made of acetonitrile/water containing 0.1 % TFA (60:40, v/v) at a flow rate of 1 mL/min in the isocratic mode at 25°C.

Results are given in Table 4 and Figure 2.

Table 4 summarizes the travoprost elution during the swelling in saline of dry travoprost-loaded MS. and the further travoprost elution in PBS (in sink condition). The travoprost loading was 1 mg/mL.

Table 4. Travoprost elution after swelling of MS loaded at 1 mg/mL and after their subsequent transfer in PBS according to example 4.

| Test number | % of travoprost release | | | | MS *in vitro* degradation time (days) |
|---|---|---|---|---|---|
| | During MS swelling | After 1 day in PBS | After 7 days in PBS | After 18 days in PBS | |
| MS1 | 12.28 ± 0.08 | 49.11 ± 2.60 | x | X | 12 |
| MS2 | 2.82 ± 0.73 | 14.6 ± 0.74 | x | X | 24 |
| MS3 | 11.5 ± 5 | 49.3 ± 5.8 | 64 ± 9.4 | 80.5 | 180 |
| MS4 | 1.71 ± 0.3 | 19.5 ± 7.54 | x | X | > 180 |
| MS5 | 1.84 ± 0.25 | 11.6 ± 1 | 17.9 | 27.4 | >>180 |

(continued)

| Test number | % of travoprost release | | | | MS *in vitro* degradation time (days) |
|---|---|---|---|---|---|
| | During MS swelling | After 1 day in PBS | After 7 days in PBS | After 18 days in PBS | |
| MS6 | 1.32 ± 0.11 | 10.9 ± 2.1 | 17.8 | 26 | >>>180 |
| *X=not measured* | | | | | |

Table 5 summarizes the travoprost elution during the swelling in saline of dry travoprost-loaded MS. and the further travoprost elution in PBS (in sink condition). The travoprost loading was 2 mg/mL.

Table 5. Travoprost elution after swelling of MS loaded at 2 mg/mL and after their subsequent transfer in PBS according to example 4.

| Test number | % of travoprost release | | | | MS *in vitro* degradation time (days) |
|---|---|---|---|---|---|
| | During MS swelling | After 1 day in PBS | After 7 days in PBS | After 18 days in PBS | |
| MS2 | 2.80 ± 0.29 | 12.74 ± 0.26 | x | X | > 180 |
| MS4 | 1.82 ± 0.15 | 15.75 ± 3.9 | x | X | > 180 |
| MS5 | 1.71 ± 0.27 | 11.8 ± 0.78 | 19.5 | 28.8 | >>180 |
| MS6 | 1.57 ± 0.11 | 9.7 ± 0.77 | 15.7 | 23.3 | >>>180 |
| *X=not measured* | | | | | |

Figure 2 represents the effect of MS composition on travoprost release during the swelling of dry microspheres in saline during 10 minutes at room temperature.
Figure 3 represents the elution of travoprost for MS3-5-6 for a travoprost loading of 1 mg/mL
Figure 4 represent the elution of travoprost for MS5-6 for a travoprost loading of 2 mg/mL

**Example 5 : Size distribution of sterile hydrophilic microspheres (MS1 εt MS3) after swelling in saline**

The sterile microspheres from two batches with crosslinker having different hydrophobicity have similar size distribution. No difference in terms of size was found according to the Mann-Whitney non-parametric test (p = 0.2493). (See Figures 5A and 5B)

## Claims

1. A composition comprising an effective amount of a prostaglandin analogue, at least one hydrophilic degradable microsphere comprising a crosslinked matrix, and a pharmaceutically acceptable carrier for administration by injection, the crosslinked matrix being based on at least:

    a) between 10 % and 90 % of hydrophilic monomer of general formula (I):

    $$(CH_2=CR_1)\text{-}CO\text{-}D \qquad (I)$$

    wherein:

    - D is O-Z or NH-Z, Z being $(C_1\text{-}C_6)$alkyl group, $-(CR_2R_3)_m\text{-}CH_3$, $-(CH_2\text{-}CH_2\text{-}O)_m\text{-}H$, $(CH_2\text{-}CH_2\text{-}O)_m\text{-}CH_3$,

-$C(R_4OH)m$ or -$(CH_2)_m$-$NR_5R_6$ with m being an integer from 1 to 30;
- $R_1$, $R_2$, $R_3$, $R_4$, $R_5$ and $R_6$ are, independently of one another, hydrogen atom or a ($C_1$-$C_6$)alkyl group;

b) between 0.1 and 30% mol of a cyclic monomer having an exo-methylene group of formula (II):

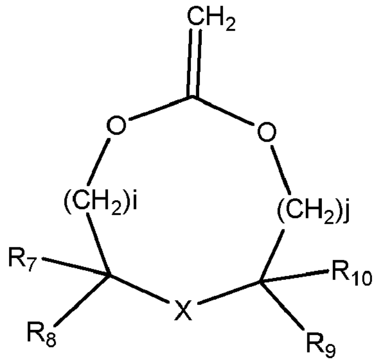

(II)

wherein:

- $R_7$, $R_8$, $R_9$ and $R_{10}$ are, independently of one another, hydrogen atom, a ($C_1$-$C_6$)alkyl group or an aryl group;
- i and j are independently of one another an integer chosen between 0 and 2; and
- X is a single bond or an oxygen atom;

and
c) between 5 % and 90 % of one degradable block copolymer cross-linker, wherein the degradable block copolymer crosslinker is linear or star-shaped and presents ($CH_2$=($CR_{11}$))-groups at all its extremities, each $R_{11}$ being independently of one another hydrogen atom or a ($C_1$-$C_6$)alkyl group,

the percentages of the monomers a) to c) being expressed in moles relative to the total number of moles of the monomers.

**2.** The composition of claim 1, wherein the degradable block copolymer cross-linker c) is of general formula (IIIa), (IIIb) or (IIIc) as follows:

$(CH_2=CR_{11})$-CO-$X_n$-$PEG_p$-$X_k$-CO-$(CR_{11}=CH_2)$ (IIIa);

$(CH_2=CR_{11})$-CO-O-$X_n$-$PEG_p$-$X_k$-O-CO-$(CR_{11}=CH_2)$ (IIIb)

$PEG_p$-($X_n$-O-CO-$(CR_{11}=CH_2))_z$ (IIIc);

wherein:

- each $R_{11}$ is independently of one another hydrogen atom or a ($C_1$-$C_6$)alkyl group;
- X independently represents, PLA, PGA, PLGA, PCL or PLAPCL;
- n, k and p respectively represent the degree of polymerization of X, and PEG, n and k independently being integers from 1 to 150, and p being an integer from 1 to 100;
- z represents the number of arms of the PEG molecule being integers from 3 to 8.

**3.** The composition of claim 2, wherein the degradable block copolymer cross-linker c) has a partition coefficient P of between 0.50 and 11.20, or a hydrophobic/hydrophilic balance R between 1 and 20.

**4.** The composition of any one of claims 1 to 3, wherein the degradable block copolymer cross-linker c) is of general formula (IIIa), (IIIb) and (IIIc), in particular (IIIa) or (IIIb) wherein X represents PCL or PLAPCL.

**5.** The composition of any one of claims 1 to 4, wherein the degradable block copolymer cross-linker c) is selected from the group consisting of compounds of general formula (IIIa), (IIIb) or (IIIc), in particular (IIIa) or (IIIb), as defined above, wherein:

- X= PLA, n + k=12 and p=13;
- X= PLAPCL, n + k=10 and p=13;
- X = PLAPCL, n + k= 9 and p=13;
- X = PLAPCL, n + k= 8 and p=13;
- X = PCL; n + k = 8 and p=13;
- X =PCL, n + k = 10 and p=4; or
- X =PCL, n + k = 12 and p=2.

**6.** The composition of any one of claims 1 to 5, wherein the degradable block copolymer cross-linker c) is present in the reaction mixture in an amount of between 5% and 60 %, advantageously between 15% and 60% by mole, relative to the total number of moles of the monomers.

**7.** The composition of any one of claims 1 to 6, wherein the cyclic monomer b) is selected from the group consisting of 2-methylene-1,3-dioxolane, 2-methylene-1,3-dioxane, 2-methylene-1,3-dioxepane, 2-methylene-4-phenyl-1,3-dioxolane, 2-Methylene-1,3,6-Trioxocane and 5,6-benzo-2-methylene-1,3dioxepane.

**8.** The composition of any one of claims 1 to 7, wherein the hydrophilic monomer a) is selected from the group consisting of sec-butyl acrylate, n-butyl acrylate, t-butyl acrylate, t-butyl methacrylate, methylmethacrylate, N-dimethyl-aminoethyl(methyl)acrylate, N,N-dimethylaminopropyl-(meth)acrylate, t-butylaminoethyl (methyl)acrylate, N,N-diethylaminoacrylate, acrylate terminated poly(ethylene oxide), methacrylate terminated poly(ethylene oxide), methoxy poly(ethylene oxide) methacrylate, butoxy poly(ethylene oxide) methacrylate, acrylate terminated poly(ethylene glycol), methacrylate terminated poly(ethylene glycol), methoxy poly(ethylene glycol) methacrylate, butoxy poly(ethylene glycol) methacrylate.

**9.** The composition of any one of claims 1 to 8, wherein the crosslinked matrix of the hydrophilic degradable microsphere is further based on a chain transfer agent d).

**10.** The composition of any one of claims 1 to 9, comprising between 1 and 6 mg/ml of a prostaglandin analogue, advantageously of travoprost.

**11.** A composition as defined in any one of claims 1 to 10, for use for preventing and/or treating ocular hypertension or glaucoma.

**12.** A composition for use according to claim 11, wherein the prostaglandin analogue is released during a period of at least 3 weeks, advantageously during a period of between 3 weeks and 6 months.

**13.** A hydrophilic degradable microsphere for use for delivering an effective amount of a prostaglandin analogue, advantageously of travoprost, to a subject in need thereof, the hydrophilic degradable microsphere being as defined in any one of claims 1 to 10.

**14.** The hydrophilic degradable microsphere for use according to claim 13, wherein the prostaglandin analogue, advantageously of the travoprost, is released during a period of at least 3 weeks, advantageously during a period of between 3 weeks and 6 months.

**15.** Pharmaceutical kit comprising:

i) at least one hydrophilic degradable microsphere as defined in anyone of claims 1 to 10 in association with a pharmaceutically acceptable carrier for administration by injection;
ii) an effective amount of a prostaglandin analogue, advantageously of travoprost; and
iii) optionally an injection device,

the hydrophilic degradable microsphere and the travoprost being packed separately.

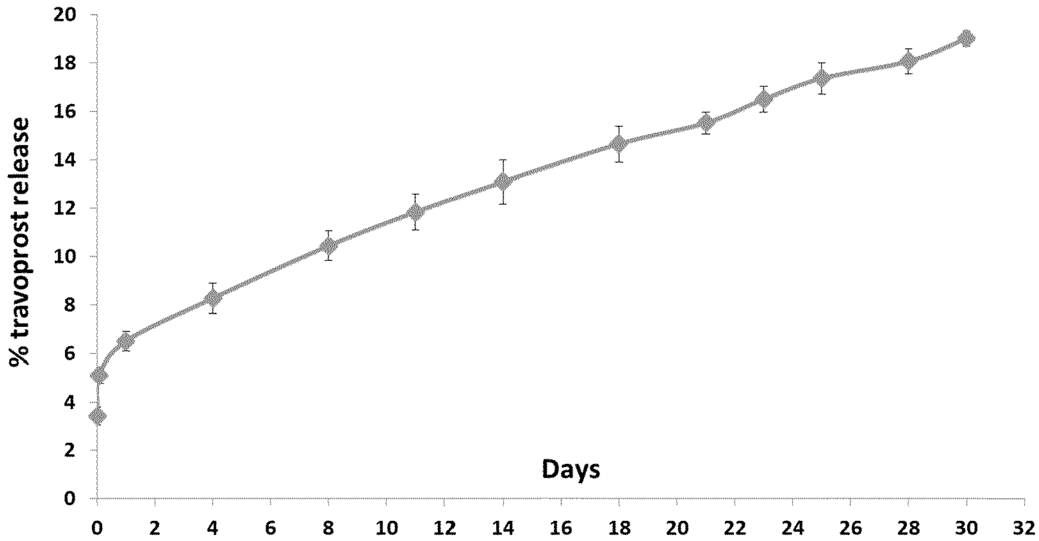
% travoprost release
Days
0
2
4
6
8
10
12
14
16
18
20
0
2
4
6
8
10
12
14
16
18
20
22
24
26
28
30
32

Fig. 1

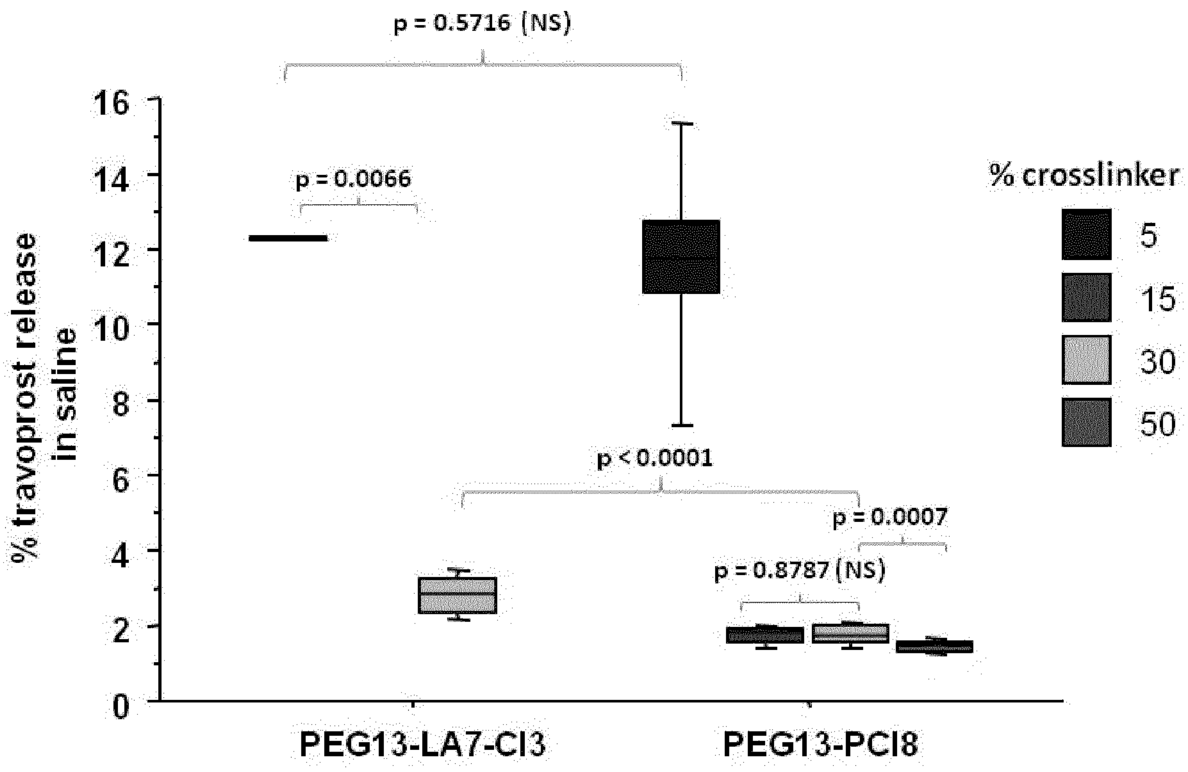
p = 0.5716 (NS)
p = 0.0066
% crosslinker
5
15
30
50
% travoprost release in saline
p < 0.0001
p = 0.0007
p = 0.8787 (NS)
0
2
4
6
8
10
12
14
16
PEG13-LA7-Cl3
PEG13-PCl8

Fig. 2

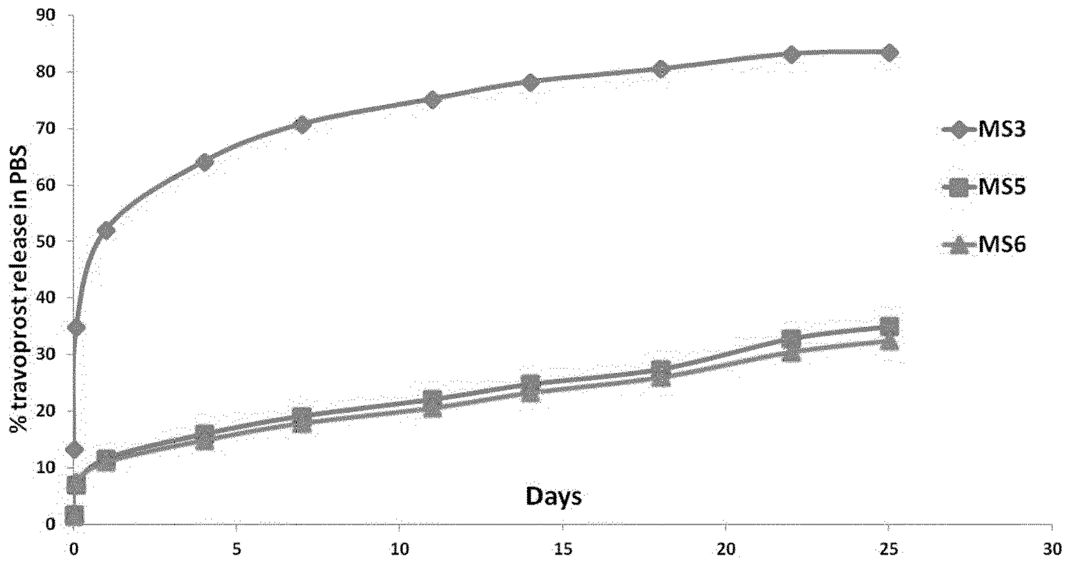
% travoprost release in PBS
Days
MS3
MS5
MS6
0
10
20
30
40
50
60
70
80
90
0
5
10
15
20
25
30

Fig. 3

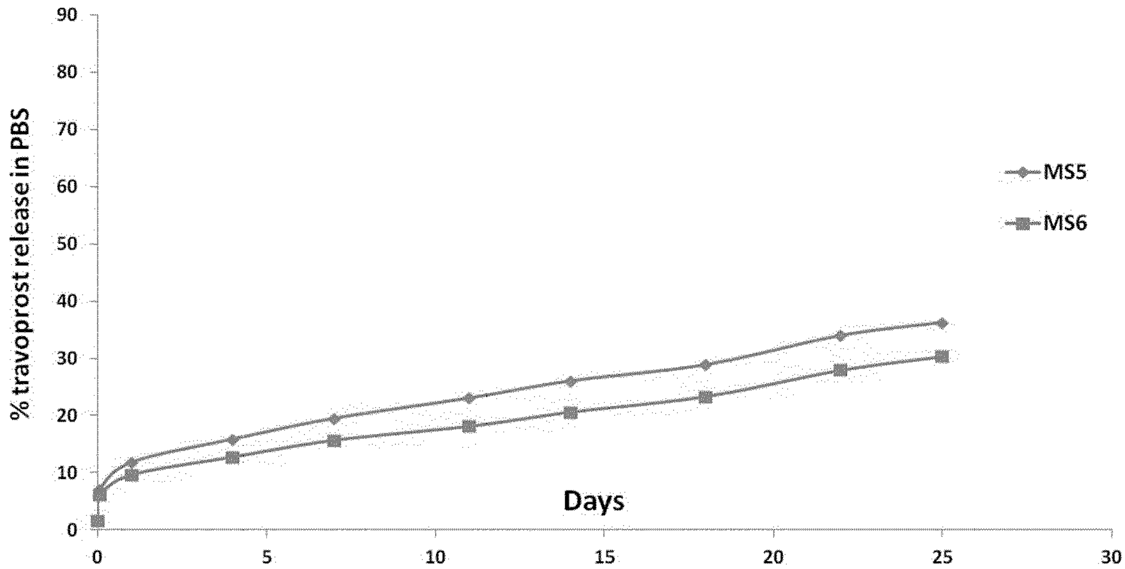
% travoprost release in PBS
Days
MS5
MS6
0
10
20
30
40
50
60
70
80
90
0
5
10
15
20
25
30

Fig. 4

MS1 :

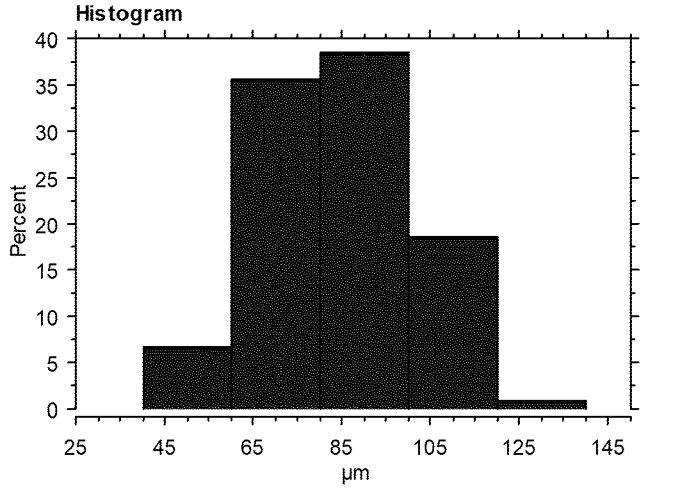


Descriptive Statistics

| | µm |
|---|---|
| Mean | 83,770 |
| Std. Dev. | 16,067 |
| Std. Error | 1,383 |
| Count | 135 |
| Minimum | 50,014 |
| Maximum | 122,096 |
| # Missing | 0 |

Fig. 5A

MS3 :

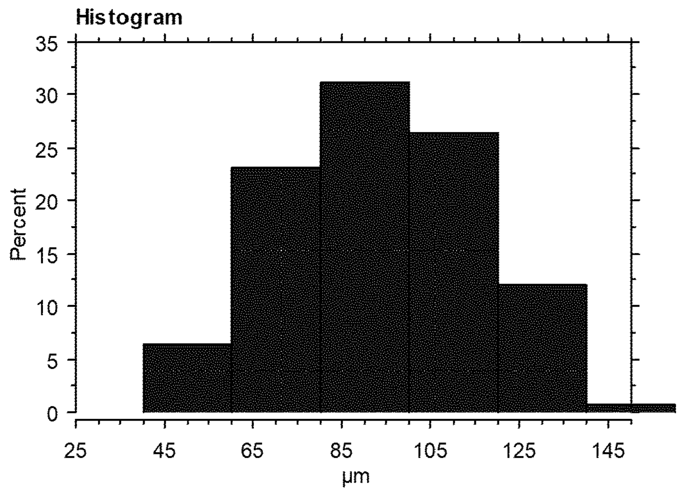


Descriptive Statistics

| | µm |
|---|---|
| Mean | 93,778 |
| Std. Dev. | 21,400 |
| Std. Error | 1,914 |
| Count | 125 |
| Minimum | 50,759 |
| Maximum | 142,899 |
| # Missing | 0 |

Fig. 5B

Europäisches Patentamt
European Patent Office
Office européen des brevets

# EUROPEAN SEARCH REPORT

Application Number
EP 20 30 5777

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2006/182781 A1 (HUGHES PATRICK [US] ET AL) 17 August 2006 (2006-08-17)<br>* claims *<br>* examples *<br>----- | 1-15 | INV.<br>A61K9/00<br>A61K9/16<br>A61K9/19<br>A61K9/50 |
| Y | WO 2012/120138 A1 (OCCLUGEL [FR]; CENTRE NAT RECH SCIENT [FR] ET AL.)<br>13 September 2012 (2012-09-13)<br>* examples *<br>* claims *<br>* page 26, lines 15-24 *<br>* page 32, line 32 *<br>----- | 1-15 | |

TECHNICAL FIELDS SEARCHED (IPC)

A61K

The present search report has been drawn up for all claims

1

| Place of search | Date of completion of the search | Examiner |
|---|---|---|
| Munich | 9 February 2021 | Gerber, Myriam |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 20 30 5777

This annex lists the patent family members relating to the patent documents cited in the above-mentioned European search report.
The members are as contained in the European Patent Office EDP file on
The European Patent Office is in no way liable for these particulars which are merely given for the purpose of information.

09-02-2021

| Patent document cited in search report | Publication date | Patent family member(s) | Publication date |
|---|---|---|---|
| US 2006182781 A1 | 17-08-2006 | NONE | |
| WO 2012120138 A1 | 13-09-2012 | AU 2012224524 A1 | 26-09-2013 |
| | | EP 2683750 A1 | 15-01-2014 |
| | | ES 2632769 T3 | 15-09-2017 |
| | | JP 6078480 B2 | 08-02-2017 |
| | | JP 2014511426 A | 15-05-2014 |
| | | US 2013344159 A1 | 26-12-2013 |
| | | WO 2012120138 A1 | 13-09-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **YADAV.** *Materials Science &t Engineering C,* 2019, 103
- **LAMBERT.** *Transl Vis Sci Technol.,* 2015, vol. 4 (1
- **NORDSTROM et al.** *Am J Ophthalmol.,* 2005, vol. 140, 598-606
- **QUEK et al.** *Arch Ophthalmol.,* 2011, vol. 129, 643-8
- **BROWN et al.** *Can J Ophthalmol.,* 1984, vol. 19, 2-5
- **SONG et al.** *J Glaucoma,* 2013, vol. 22, 190-4
- **GANDOLFI et al.** *Arch Ophthalmol.,* 1995, vol. 113, 582-5
- **SEN et al.** *Br J Ophthalmol.,* 2005, vol. 89, 917-8
- **CARBONNIÈRE et al.** *J Fr Ophtalmol.,* 2017, vol. 40, 177-86
- **ZHANG et al.** *Invest Ophthalmol Vis Sci.,* 2011, vol. 52, 7787-91
- **SUBRIZI et al.** *Drug Discovery Today,* 2019, vol. 24, 1446-57
- **NG et al.** *Drug Deliv. and Transl. Res.,* 2015, vol. 5, 469-79
- **LAVIK et al.** *J Ocul Pharmacol Ther.,* 2016, vol. 32, 642-49
- **HUANG et al.** *J Ocul Pharmacol Ther.,* 2005, vol. 21, 445-53
- **FEDORCHAK et al.** *Exp Eye Res.,* August 2014, vol. 125, 210-6
- **SALAMA et al.** *AAPS PharmSciTech.,* 2017, vol. 18, 2517-28
- **GIARMOUKAKIS et al.** *Exp Eye Res.,* 2013, vol. 112, 29-36
- **NATARAJAN et al.** *ACS Nano,* 2014, vol. 8, 419-29
- **SHU et al.** *Eye and Vision,* 2019, vol. 6, 15
- **LI et al.** *Medicine,* 2018, vol. 97, 17
- **BENZON et al.** *Anesthesiology,* 2007, vol. 106, 331-8
- **MARTINEZ-DE-LA-CASA et al.** *Eye,* 2012, vol. 26, 972-75